# EUROPEAN PATENT APPLICATION

(11) **EP 3 805 776 A1**
(43) Date of publication of application: **14.04.2021**
(21) Application number: 19201724.2
(22) Date of filing: 07.10.2019
(51) Int. Cl.: G01R 33/58, G01R 33/54, A61B 5/055, A61B 5/00, G06N 3/08, G01R 33/561, G01R 33/565

(54) **SYSTEM AND METHOD FOR DETERMINING OPTIMAL RESONANCE FREQUENCY SETTING OF A MAGNETIC RESONANCE DEVICE**

(71) Applicant: Siemens Healthcare GmbH, 91052 Erlangen (DE)
(72) Inventor: Hayes, Carmel, 80634 München (DE)

(57) **Abstract**

The invention provides a method and a system for operating a magnetic resonance imaging, MRI, device, (1). The system (100) comprises: an input interface (10) for receiving a series (71) of magnetic resonance, MR, images obtained at the same slice position with different resonance frequency setting values, RFSV;
a computing device (50) configured to implement a trained machine learning algorithm, MLA, (55) wherein the trained MLA (55) is trained to receive the series (71) of MR images received by the input interface (10) as its input and to generate, based thereon, as its output a recommendation signal (73) indicating an optimal RFSV; and
an output interface (90) for outputting the recommendation signal (73).

## Description

The present invention relates to a system and a method for operating a magnetic resonance device. Moreover, the invention relates to a method for training a machine learning algorithm, in particular for use in said system and/or said method. The invention further relates to computer program products and data storage media comprising program code.

Magnetic resonance, MR, imaging at comparatively high field strengths, e.g. larger than 1.5 Tesla, often requires additional adjustments steps in order to ensure that an image quality of the generated MR images is of sufficient quality for clinical usage. For example, artefacts associated with B0 inhomogeneities, wherein B0 is the usual designation for the main static magnetic field generated by a magnetic resonance imaging, MRI, device (or: MR imaging scanner, or: MR device).

For example, when imaging the heart using steady-state coherent sequences in which balanced gradients are used along all three spatial axes (such as the TrueFISP sequence (trademarked)), it is often very beneficial to shim a region within the image that includes the heart and great blood vessels, wherein "shimming" refers to methods for homogenizing the B0 magnetic field. "FISP" therein stands for "Fast Imaging with Steady-state Precession".

"Balanced" in this context may in particular mean that the net gradient-induced dephasing over a TR (repetition time) interval is zero. Conversely, some of the gradients in FISP may be unbalanced, allowing recording of separate FID (Free Induction Decay) and echo components of the steady-state free precession signal. In True FISP, however, the balanced gradients refocus both components at the exact center of the TR interval as a single echo.

Usually a so-called pre-scan (or: TrueFISP frequency scout (trademarked)) is also performed in order to further refine the choice of the optimal imaging frequency within the heart. This pre-scan generates a series of images (pre-scan images) taken at one particular given slice location each with a different resonance frequency setting value for the MRI device.

Typically, a user then reviews the pre-scan images manually so as to identify the image (and, accordingly, the corresponding resonance frequency setting value) which best fulfils given quality criteria. The main scan following the pre-scan is then performed using that resonance frequency setting value for the actual MR imaging (or: MR scan). Evidently, which quality criteria are applied strongly depends on the intended scan to be performed, on the slice position (i.e. the position of the scan with respect to the patient's body) and/or the like. For example, the number of artefacts, the contrast and/or the like may factor into the quality criteria.

One particular workflow may be implemented as follows: a user first identifies the location of the heart within the thorax and places a shim box over the heart. Then, localizers are run to assess image quality and to localize the heart's axes. After that, a pre-scan (e.g. a TrueFISP frequency scout) is performed at one slice location within the heart. The pre-scan images are manually inspected by a user and the best suited image, and thus the most suitable, or at least the most promising resonance frequency setting value is selected by the user. Said resonance frequency setting value is noted either mentally or on paper, and then entered into subsequent scanning protocols (i.e. parameter settings for the MR scan) as a parameter.

Artefact reduction using TrueFISP is described, for example, in the scientific publication by V. Despande et al., "Artifact Reduction in TrueFISP Imaging of the Coronary Arteries by Adjusting Imaging Frequency", in : Magnetic Resonance in Medicine 49:803-809 (2003), DOI 10.1002/mrm.10442.

It is one of the objects of the present invention to provide methods and systems for operating a magnetic imaging device faster and more reliably. This object is solved by the subject-matter of the independent claims. Further advantageous embodiments and variants are expressed by the dependent claims.

In particular, the invention provides according to a first aspect a system for operating a magnetic resonance imaging, MRI, device, comprising:
an input interface for receiving a series of magnetic resonance, MR, images obtained at the same slice position with different resonance frequency setting values, RFSV; a computing device configured to implement a trained machine learning algorithm, MLA, wherein the trained MLA is trained to receive the series of MR images received by the input interface as its input and to generate, based thereon, as its output a recommendation signal indicating an optimal RFSV; and an output interface for outputting the recommendation signal.

Whenever in the present context an MR image, or a series of MR images, is mentioned, it shall be understood that these image each correspond to one particular resonance frequency setting value, RFSV, and that the images are to be understood as comprising the information to which resonance frequency setting value, RFSV, they correspond. Thus, when a particular MR image is selected as "the best", i.e. as best fulfilling given quality criteria, this concurrently means that a corresponding resonance frequency setting value, RFSV has been selected as optimal. Indicating an MR image with the recommendation signal is thus the same as indicating a resonance frequency setting value, RFSV.

In particular, the system may be used for operating a magnetic resonance imaging, MRI, device for a cardiac MR scan.

The resonance frequency setting value, RFSV, may in particular be a value for a setting of a resonance frequency of a substance present in the human body, in particular of water. Such a resonance frequency setting value, RFSV, is often expressed as an offset (such as - 200Hz or +40 Hz) to a predefined absolute frequency setting value (e.g. corresponding to an absolute resonance frequency value of water), but may alternatively also be expressed as an absolute value itself.

The input interface and/or the output interface may be realized in hardware and/or software. The input interface and/or the output interface can each comprise one or more different communication channels using one or more different communication protocols (e.g. HTTP). Each of the input interface and/or the output interface can be configured to connect to a cable-bound data connection and/or to a wireless data connection such as Bluetooth, Zigbee, WiFi and so on. The input interface and/or the output interface may also be configured to connect to Ethernet networks.

The input interface may be connected to a Picture Archiving and Communications System (PACS) which may in some variants also be part of the system according to the first aspect of the invention. The input interface may be configured to receive the series of MR images from the PACS and may also be configured to request the sending of the series by the PACS to the input interface from the PACS.

The computing device may be realized as any device, or any means, for computing. For example, the computing device may comprise at least one data processing (or: calculating) unit such as at least one central processing unit, CPU, and/or at least one graphics processing unit, GPU, and/or at least one field-programmable gate array, FPTA, and/or at least one application-specific integrated circuit, ASIC, and/or any combination of the foregoing. The computing device may comprise a working memory operatively coupled to the at least one processing unit and/or a non-transitory memory operatively connected to the at least one processing unit and/or the working memory.

The computing device may be partially or completely realized as a remote device, for example as a cloud computing platform.

The term "the same slice position" should be understood to mean in particular that the MR images of the series have been taken at the same portion ("slice") of the same patient's body (using different resonance frequency setting values, RSFV), small movements or measurement deviations notwithstanding.

The series of MR images may comprise as little as two different images but will usually comprise at least three, preferably at least four, more preferably at least five or even more images. It is also preferred that the MR images of the series are taken with respective resonance frequency setting values, RFSV, which are separated from one another by a fixed amount, such as a difference of 10 Hz, of 20Hz or 40Hz between individual MR images.

One of the advantages of the present invention is that the machine learning algorithm, MLA, is generally able to distinguish more reliably between even very similar-looking MR images than it is possible with the human eye, and to do it even faster. Thus, with the present invention, the frequency value difference between the individual MR images may be chosen smaller than it would be for a human because the machine learning algorithm, MLA, is actually able to differentiate between the MR images and so the optimal resonance frequency setting value, RFSV, may be determined by the machine learning algorithm, MLA, not only more accurately but also more precisely.

For example, a series of MR images ranging from offset values for the predetermined resonance frequency setting value for water of between -150 Hz and 150 Hz at steps of 25 Hz may be used.

Another advantage of using a trained (and thus trainable) machine learning algorithm, MLA, is that the machine learning algorithm, MLA, be continuously improved and updated. In this way, over time, even series of MR images that are exceedingly noisy (i.e. comprise a lot of artefacts) can be treated by the machine learning algorithm.

In some advantageous embodiments, variants, or refinements of embodiments, the system further comprises an MRI device. The output interface may be configured to output the recommendation signal to the MRI device. The recommendation signal may be configured to adjust a parameter setting of the MRI device. Specifically, the recommendation signal may be configured to adjust a resonance frequency setting of the MRI device to the one indicated by the recommendation signal. In other words, the recommendation signal may indicate the resonance frequency setting value, RFSV, in the sense that it sets the parameter setting of the MRI device to that value. It will be understood that in the case that the resonance frequency setting value, RFSV, indicates offset values, the MRI device will be set to the predefined resonance frequency value, modified by the offset value given by the resonance frequency setting value, RFSV.

However, alternatively, the recommendation signal may also be configured to control a display to display the indicated (i.e. recommended) resonance frequency setting value, RFSV, to a user so that the user may input that value into the MRI device themselves. Yet alternatively, the recommendation signal may be configured to control a graphical user interface to display the indicated (or: recommended) resonance frequency setting value, RFSV, to a user for confirmation (upon which the resonance frequency setting of the MRI device may be adjusted accordingly) or for dismissal or adjustment. In case of dismissal and/or adjustment, the user may be presented automatically with the series of MR images to determine the optimal resonance frequency setting value, RFSV, themselves.

In some advantageous embodiments, variants, or refinements of embodiments, the trained MLA is further configured and trained to receive, as a further input, at least one additional piece of information, and to generate the recommendation signal based also on said at least one additional piece of information. Thus, additional pieces of information that are known to improve the decision on the optimal resonance frequency setting value, RFSV, may be added to the input so as to improve the precision and/or accuracy of the output of the machine learning algorithm, MLA.

For example, the at least one additional piece of information may be a piece of information indicating a location of an organ of interest within the input series. Since the present invention is especially applicable to the field of cardiac MR imaging, said organ of interest may in particular be a heart, preferably a human heart.

In some advantageous embodiments, variants, or refinements of embodiments, the trained machine learning algorithm, MLA, is a trained artificial neural network. Such networks have been found by the inventor to be very efficient for the tasks described herein and to be yet easily trainable for that purpose. In particular, the trained machine learning algorithm, MLA, may be a trained artificial deep neural network, i.e. a trained artificial neural network with more than one hidden layer between an input layer and an output layer.

According to a second aspect, the invention further provides a computer-implemented method for operating a magnetic resonance imaging, MRI, device, comprising: receiving a series of MR images obtained at the same slice position with different resonance frequency setting values, RFSV;
providing a trained machine learning algorithm, MLA, configured and trained to receive, as an input, such a series of MR images, and further configured and trained to generate, as an output, a recommendation signal indicating an optimal RFSV based at least on said input;
inputting the received series of MR images into the provided trained machine learning algorithm, MLA;
generating, by the provided trained MLA, the recommendation signal based on the input series; and
outputting the recommendation signal.

In some advantageous embodiments, variants, or refinements of embodiments, the method comprises a step of adjusting a parameter setting of the MRI device based on the recommendation signal. As has been described in the foregoing, said parameter setting of the MRI device may in particular be a setting for the resonance frequency of water. The step of adjusting may be conditional upon approval of a user, but is preferably performed automatically so that human supervision becomes unnecessary.

According to a third aspect of the present invention, a computer program product is provided which comprises executable program code configured to, when executed, perform the method according to any embodiment of the second aspect of the present invention.

According to a fourth aspect of the present invention, a non-transitory, computer-readable data storage medium is provided which comprises executable program code configured to, when executed, perform the method according to any embodiment of the second aspect of the present invention.

According to a fifth aspect of the present invention, a data stream is provided, which comprises program code (or which is configured to generate program code), configured to, when executed by a computing device, perform the method according to any embodiment of the second aspect of the present invention. The data stream may, for example, be provided by a server and be downloaded by a client or a user terminal or by a personal computer or a laptop.

According to a sixth aspect, the invention further provides a computer-implemented method for training a machine learning algorithm, MLA, comprising:
providing training samples, wherein each training sample comprises a series of MR images obtained at the same slice position for at least two different resonance frequency setting values and each training sample further comprises a label indicating which image of the series of images best fulfils a given quality criterion;
providing a machine learning algorithm, MLA, configured to receive said training samples as an input and to output based thereon a recommendation signal indicating an optimal RSFV value, wherein the machine learning algorithm, MLA, comprises trainable parameters;
training the MLA by updating the trainable parameters based on a loss function penalizing differences between an output of the machine learning algorithm, MLA, for a given training sample and the label of the same training sample.

The difference may e.g. be a difference between the resonance frequency setting value, RFSV, indicated by the recommendation signal and a corresponding label, or it may be a difference between an image corresponding to said indicated value and an image according to the label.

The trainable parameters may in particular be weights and biases of an artificial neural network but may also comprise other parameters such as filter sizes for convolutional neural networks and/or the like.

In some advantageous embodiments, variants, or refinements of embodiments, the machine learning algorithm, MLA, is further configured to receive, as a further input, at least one additional piece of information, and to generate the output based also on said at least one additional piece of information, wherein each training sample further comprises the at least one additional piece of information. Thus, additional pieces of information that are known to improve the decision on the optimal resonance frequency setting value, RFSV, may be added to the input so as to improve the precision and/or accuracy of the output of the machine learning algorithm, MLA.

For example, the at least one additional piece of information may be a piece of information indicating a location of an organ of interest within the input series. Since the present invention is especially applicable to the field of cardiac MR imaging, said organ of interest may in particular be a heart, preferably a human heart.

According to a seventh aspect of the present invention, a computer program product is provided which comprises executable program code configured to, when executed, perform the method according to any embodiment of the fifth aspect of the present invention.

According to an eighth aspect of the present invention, a non-transitory, computer-readable data storage medium is provided which comprises executable program code configured to, when executed, perform the method according to any embodiment of the fifth aspect of the present invention.

According to a ninth aspect of the present invention, a data stream is provided, which comprises program code (or which is configured to generate program code), configured to, when executed by a computing device, perform the method according to any embodiment of the sixth aspect of the present invention. The data stream may, for example, be provided by a server and be downloaded by a client or a user terminal or by a personal computer or a laptop.

Additional advantageous variants, refinements, embodiments and aspects of the invention will become more obvious in connection with the following description with reference to the drawings.

### Brief Description of the Drawings

The invention will be explained in yet greater detail with reference to exemplary embodiments depicted in the drawings as appended.

The accompanying drawings are included to provide a further understanding of the present invention and are incorporated in and constitute a part of the specification. The drawings illustrate the embodiments of the present invention and together with the description serve to illustrate the principles of the invention. Other embodiments of the present invention and many of the intended advantages of the present invention will be readily appreciated as they become better understood by reference to the following detailed description. Like reference numerals designate corresponding similar parts.

The numbering of method steps is intended to facilitate understanding and should not be construed, unless explicitly stated otherwise, or implicitly clear, to mean that the designated steps have to be performed according to the numbering of their reference signs. In particular, several or even all of the method steps may be performed simultaneously, in an overlapping way or sequentially.

In the drawings:
- Fig. 1: shows a schematic block diagram illustrating a system according to an embodiment of the first aspect of the present invention;
- Fig. 2: shows a schematic flow diagram illustrating a computer-implemented method according to an embodiment of the second aspect of the present invention;
- Fig. 3: shows a schematic flow diagram illustrating a computer-implemented method according to an embodiment of the sixth aspect of the present invention;
- Fig. 4: shows a schematic block diagram illustrating a computer program product according to an embodiment of the third aspect of the present invention or according to an embodiment of the sixth aspect of the present invention; and
- Fig. 5: shows a schematic block diagram illustrating a data storage medium according to an embodiment of the fourth aspect of the present invention or according to an embodiment of the eighth aspect of the present invention.

Although specific embodiments have been illustrated and described herein, it will be appreciated by those of ordinary skill in the art that the variety of alternate and/or equivalent implementations may be substituted for the specific embodiments shown and described without departing from the scope of the present invention. Generally, this application is intended to cover any adaptations or variations of the specific embodiments discussed herein.

### Detailed Description of the Invention

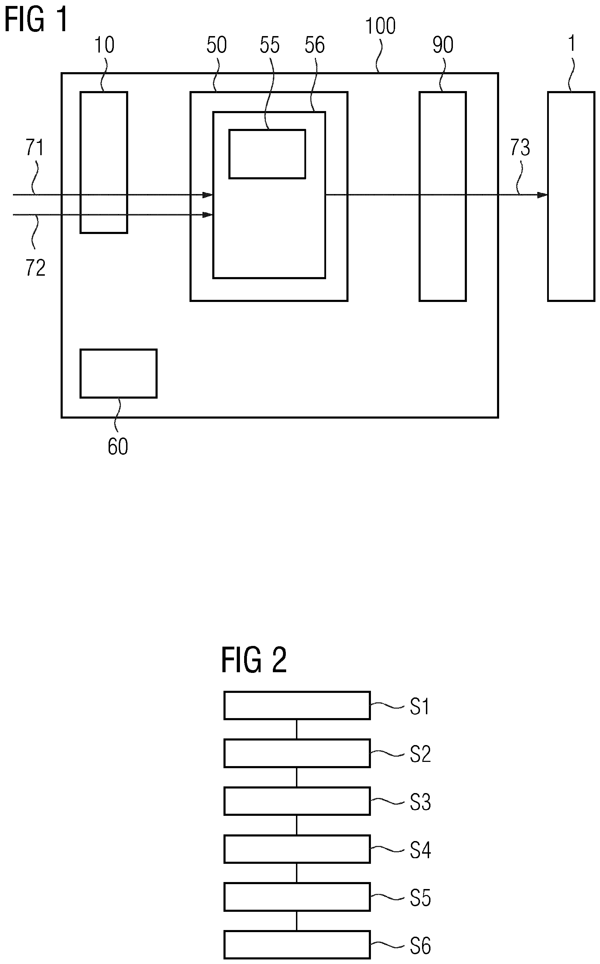

Fig. 1 shows a schematic block diagram illustrating a system 100 according to an embodiment of the first aspect of the present invention. Thus, Fig. 1 shows a schematic block diagram of a system 100 for providing a recommendation signal 73, for use in a magnetic resonance, MR, scan performed by an magnetic resonance imaging, MRI, device 1. The MRI device 1 may be part of the system 100 or may be separate from it.

The system 100 further comprises an input interface 10 for receiving a series 71 of magnetic resonance, MR, images obtained at the same slice position with different resonance frequency setting values, RFSV.

The system 100 also comprises a computing device 50 configured to implement a trained machine learning algorithm, MLA 55. The computing device 50 may comprise a dedicated machine learning algorithm, MLA, module 56 configured to implement the trained MLA 55. The MLA module 56 may in particular be realized as software run by the computing device 50, wherein said software may be stored in a non-transitory data storage of the computing device 50 and may be run in a working memory of the computing device 50.

The trained MLA 55 is trained to receive the series 71 received by the input interface 10 as its input and to generate, as its output, the recommendation signal 73, wherein the recommendation signal 73 indicates an optimal resonance frequency setting value, RFSV. Preferably, the resonance frequency setting value, RFSV, refers to an offset (or alternatively to an absolute value) for a frequency setting of the MRI device 1 for the resonance frequency of water.

The system 100 further comprises an output interface 90 for outputting the recommendation signal 73. The generated recommendation signal 73 may be output e.g. to an external database for storing therein, preferably together with the corresponding series 71, for example for further training pre-trained machine learning algorithms, MLAs, in the future. The recommendation signal 73 may also be output to a temporary data storage.

The trained machine learning algorithm, MLA, 55 is preferably a feed-forward artificial neural network, ANN which is trained to receive as its input at least the series 71 of MR images and to generate based at least thereon the recommendation signal 73. For example, the number of images in the series 71 of images may be set as fixed to a number k, and an output layer of the machine learning algorithm, MLA, 55 realized as an artificial neural network, ANN, may comprise a softmax function. Thus, the recommendation signal 73 may output an k-dimensional vector with entries summing up to One, wherein each entry i of the vector gives the probability that the i-th image of the series 71 corresponds to the optimal resonance frequency setting value, RFSV. The one entry i with the highest value thus indicates the optimal resonance frequency setting value, RFSV for that particular series 71 of MR images.

When the machine learning algorithm, MLA, 55 is realized as an artificial neural network, ANN, the artificial neural network, ANN, may comprise be a feed-forward artificial neural network, ANN and/or a deep neural network, i.e. an artificial neural network, ANN, comprising at least one hidden layer. The feed-forward artificial neural network may be a fully connected artificial neural network comprising preferably from four to ten hidden layers, more preferably from four to six hidden layers. Each of the hidden layers may comprise from ten to one thousand neurons, preferably from fifty to two hundred neurons. In other embodiments, the artificial neural network may comprise at least one convolutional neural sub-network or may consist of a convolutional neural network.

The machine learning algorithm, MLA, 55 may further be configured to receive as its input at least one piece of additional information 72, in particular a piece of information relating to a particular series 71 of MR images. For example, the machine learning algorithm, MLA, 55 may further be trained and configured to receive information 72 about a location of an organ of interest within the input series 71. Since the present invention is especially applicable to the field of cardiac MR imaging, said organ of interest may in particular be a heart, preferably a human heart.

In case that the artificial neural network, ANN, has been trained to receive the at least one piece of additional information 72 and in the further case that for a specific resonance frequency spectrum, RFS, during the inference phase (i.e. when the trained artificial neural network, ANN, is used to make predictions based on previously unknown series 71), said piece of additional information 72 is not available for a particular series 71, an average or standardized value for said additional piece of information 72 may be used in order to allow the artificial neural network, ANN, 55 to generate the recommendation signal 73.

The system 100 may also be configured to receive, via the input interface 10, together with the resonance frequency spectrum, RFS, 71 said at least one piece of additional information 72 and/or a series identifier information, which indicates, preferably in a coded way, the corresponding series 71. In cases in which only said series identifier information, but not the at least one piece of additional information 72 is provided, the at least one piece of additional information 72 desired may be automatically requested by the system 100. For example, the output interface 90 could be configured to request the necessary information from a database (such as a PACS) based on the series identifier information. When at least part of the system 100, on particular the computing device 50, is at least partially realized as a cloud computing device, it is preferred that any information that relates to particular patients is only transmitted in an encrypted way.

The system 100 may further comprise a user interface 60, for example a touch-screen interface implementing a graphical user interface, a computer monitor in combination with a keyboard and/or a mouse, a speech-controlled user interface and/or the like. The user interface 60 is preferably configured to obtain, at least in a revision mode of the system, revision information by a user. The user may put the system 100 into the revision mode or the system 100 may permanently be in the revision mode. The revision information indicates a series 71 for which the trained machine learning algorithm, MLA, 55 has generated the recommendation signal 73 as well as indicate a confirmation the resonance frequency setting value, RFSV, indicated by the generated recommendation signal 73 and/or a correction to said indicated resonance frequency setting value, RFSV. Preferably, the revision information comprises a confirmation or correction of the indicated resonance frequency setting value, RFSV.

For example, in the revision mode, after the recommendation signal 73 has been generated, the resonance frequency setting value, RFSV, indicated by it may be visually displayed to a user by the user interface 60 together with one, several or all of the series 71 of MR images, and the user may be prompted to confirm or correct the resonance frequency setting value, RFSV, indicated by the recommendation signal 73. For example, the user interface 60 may be configured to display only the image corresponding to (i.e. that has been generated using) the resonance frequency setting value, RFSV, indicated to be optimal by the recommendation signal 73. Alternatively, the user interface 60 may be configured to display a predefined number (e.g. 3) of MR images with the highest probabilities to correspond to the actually optimal resonance frequency setting value, RFSV. The highest probabilities may be determined by the highest values of the vector output by the softmax function described above. Again alternatively, the user interface 60 may be configured to display all MR images with probabilities within a predefined range below the highest probability. In this way, the user may be shown the most relevant MR images between which the machine learning algorithm, MLA, had the most trouble identifying the one corresponding to the actually optimal resonance frequency setting value, RFSV.

For example, when a highest probability of 25% has been determined for MR image number 3, and when the predefined range is a range of 5 percentage points, then the user interface 60 may display to the user also the MR image number 5 (probability of 22%) and the MR image number 9 (probability of 21%) but none of the other MR images which have probabilities of lower than 20%.

The MR image corresponding to the resonance frequency setting value, RFSV indicated by the generated recommendation signal 73 may be automatically visually depicted so that the user can immediately verify if said MR image is indeed the one best fulfilling predefined quality criteria. The user interface 60 may provide the user with the option to compare said MR image to other MR images (e.g., as has been described in the foregoing, to others with similarly high probabilities for corresponding to the optimal resonance frequency setting value, RFSV), and to mark another MR image as being, according to the user, the one corresponding to the optimal resonance frequency setting value, RFSV. When the user does so, the recommendation signal 73 may be concurrently modified/corrected according to the settings made by the user. The recommendation signal 73 modified in this way may then be output, for example o the MRI device 1 for controlling the MRI device 1.

The finally confirmed/corrected parameter set may also be automatically be transmitted to a training entity, for example for additional training of the machine learning algorithm, MLA 55.

Fig. 2 shows a schematic flow diagram schematically illustrating a computer-implemented method for operating a magnetic resonance imaging, MRI, device. This method may in particular be performed using the system according to any embodiment of the first aspect of the present invention, in particular using the system 100 as has been described with respect to Fig. 1. It should be understood, however, that the method can also be performed independently from said system or systems.

In a step S1, a series 71 of MR images obtained at the same slice position with different resonance frequency setting value, RFSV, is received, for example by the input interface 10 of the system 100. As has been described in the foregoing, also additional pieces of information 72 may be received, including information about a location of an organ of interest (e.g. a human heart) within the input series 71.

Obtaining the series 71 of MR images may comprise, or consist of, generating the series 71 by performing, using the MRI device 1, MR scans of the same slice position (or: of the same slice, or: of the same position) with different resonance frequency setting values, RFSV. This may in particular include controlling the MRI device 1 to perform said scans. Alternatively, the series 71 of MR images may be obtained by receiving a pre-recorded series 71 of MR images from a data repository such as a PACS.

In a step S2, a trained machine learning algorithm, MLA, 55 is provided which is configured and trained to receive, as an input, such a series 71 of MR images, in particular in the sense that the dimensions and data structure of the series 71 for which the machine learning algorithm, MLA 55 has been trained match the dimensions and data structure of the series 71 received in step S1. The machine learning algorithm, MLA, 55 is further configured and trained to generate, as an output, a recommendation signal 73 indicating an optimal RFSV based at least on said input.

The machine learning algorithm, MLA, 55 is preferably a trained artificial neural network, ANN, 55 as has been described in the foregoing. In particular, the artificial neural network, ANN, may be a feed-forward artificial neural network trained with series 71 of MR images as training input, wherein the series 71 have been labeled with the corresponding recommendation signal (or with the MR image within the series which is to be identified by the MLA 55 as corresponding to the optimal resonance frequency setting value, RFSV) as ground truth.

In a step S3, the received series 71 of MR images is input into the provided trained machine learning algorithm, MLA 55. Thus, in a step S4, by the provided trained MLA 55, the recommendation signal 73 is generated based on the input series 71. In a step S5, the recommendation signal 72 is output, e.g. via the output interface 90.

In an optional step S6, a parameter setting of the MRI device 1 is adjusted based on the recommendation signal 73, for example as has been described in the foregoing. For instance, the frequency setting for a resonance frequency of a substance (in particular of water) of the MRI device 1 may be set to the resonance frequency setting value, RSFV, indicated by the recommendation signal.

In case that the system 100 is integrated into an MRI device 1, the input interface 10 and the output interface 90 may be internal interfaces within the MRI device 1 itself. The input interface 10 and the output interface 90 may even be solely realized as software interfaces, for example, when the MRI device 1 comprises a single computing device 50, which is, among others, used to implement the trained machine learning algorithm, MLA 55. The system 100 may in this case also implement other modules, for example an MRI control module, which requires the generated recommendation signal 73 for its accurate operation. The input interface 10 and the output interface 90 may in that case be software interfaces between the machine learning algorithm module 56 implemented by the computing device 50 and the MR scan control module also implemented by the computing device 50.

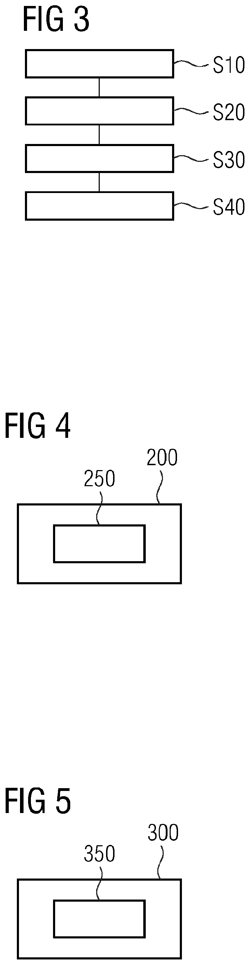

Fig. 3 shows a flow diagram schematically illustrating a computer-implemented method for training a machine learning algorithm, MLA, 55 in particular an artificial neural network, ANN, for use in a method or a system for operating a magnetic resonance imaging, MRI, device 1. In particular, the method illustrated with respect to Fig. 3 may be used for training a machine learning algorithm, MLA, 55 for use in a system 100 of Fig. 1 and/or for use with the method described with respect to Fig. 2.

In a first step S10, the machine learning algorithm, MLA, 55 is trained with labeled series 71 of MR images. The series 71 used for training may be labeled with the correct MR image that corresponds to the optimal resonance frequency setting value, RFSV. For example, when then series 71 comprises ten input images, and the MR image number 3 is the "best" MR image, i.e. it best fulfils predefined quality criteria (lack of artefacts, contrast and/or the like), the series 71 may be labelled with a ground truth vector indicating said MR image number 3, for example by a one-hot vector with a "1" as its third entry. Since each MR image is obtained with a different resonance frequency setting value, RFSV, the ground truth vector thus also indicates which resonance frequency setting value, RFSV is the optimal one. The machine learning algorithm, MLA 55 will thus learn to identify the "best" image in each new series 71 during the inference stage and to then indicate within the recommendation signal 73 the resonance frequency setting value, RFSV, corresponding to that MR image.

Alternatively, the artificial neural network of the machine learning algorithm, MLA, 55 may be trained to identify, or choose, the "best" MR image, and a following (e.g. even untrainable) algorithm within the machine learning algorithm, MLA, 55 may use a look-up table or the like to identify the resonance frequency setting value, RFSV, corresponding to the identified MR image.

In an optional step S20, the machine learning algorithm is additionally and simultaneously with step S10 trained with, as additional input, at least one piece additional information 72. For example, each training sample for the machine learning algorithm, MLA, 55 may comprise, or consist of, the series 71 of MR images and at least one piece of additional information 72 (for example location of the slice), together with the label indicating the ground truth.

It is one of the specific advantages of this invention that whenever users confirm and/or correct the resonance frequency setting value, RFSV, indicated by the recommendation signal 73 generated by the trained machine learning algorithm, MLA, 55 during the inference phase, the corrected and/or confirmed recommendation signal 73 (or another signal indicating the corrections to the resonance frequency setting value, RFSV) may be transmitted, together with the corresponding (or, in other words: underlying) series 71 to a training entity as revision information.

In a step S30, such revision information may be received by the training entity.

In a step S40, the training entity may further train the machine learning algorithm, MLA, 55 previously pre-trained in steps S10 and S20, based on the received S30 revision information, i.e. the series 71 input in the inference phase together with their corrected/confirmed recommendation signals 73 or corrected/confirmed resonance frequency setting values, RFSVs. If the machine learning algorithm, MLA, 55 is configured such as to receive, as its input, at least one additional piece of information 72, the revision information also comprises this at least one piece of information 72 for the training entity.

The training entity may be a computer program configured to further train the machine learning algorithm, MLA, 55 based on the revision information. Thus, according to another aspect, the invention also provides a training entity for performing the method according to any embodiment of the third aspect of the present invention, especially the method of Fig. 3.

Fig. 4 shows a schematic block diagram illustrating a computer program product 200 according to an embodiment of the third aspect of the present invention. The computer program product 200 comprises executable program code 250 configured to, when executed by a computing device (e. g. computing device 50 of system 100), to perform the method according to Fig. 2. Alternatively or additionally, the computer program product 200 may comprise executable program code 250 configured to, when executed by a computing device (e. g. computing device 50 of system 100), to perform the method according to Fig. 3.

Fig. 5 shows a schematic block diagram illustrating a non-transitory, computer-readable data storage medium 300 comprising executable program code 350 configured to, when executed by a computing device (such as computing device 50 of system 100), to perform the method according to Fig. 2. Alternatively or additionally, the data storage medium 300 may comprise executable program code 350 configured to, when executed by a computing device (e. g. computing device 50 of system 100), to perform the method according to Fig. 3.

In the foregoing detailed description, various features are grouped together in the examples with the purpose of streamlining the disclosure. It is to be understood that the above description is intended to be illustrative and not restrictive. It is intended to cover all alternatives, modifications and equivalence. Many other examples will be apparent to one skilled in the art upon reviewing the above specification, taking into account the various variations, modifications and options as described or suggested in the foregoing.

## Claims

1. A system (100) for operating a magnetic resonance imaging, MRI, device, (1) comprising:
an input interface (10) for receiving a series (71) of magnetic resonance, MR, images obtained at the same slice position with different resonance frequency setting values, RFSV;
a computing device (50) configured to implement a trained machine learning algorithm, MLA, (55) wherein the trained MLA (55) is trained to receive the series (71) of MR images received by the input interface (10) as its input and to generate, based thereon, as its output a recommendation signal (73) indicating an optimal RFSV; and
an output interface (90) for outputting the recommendation signal (73).

2. The system (100) of claim 1,
further comprising an MRI device (1);
wherein the output interface (90) is configured to output the recommendation signal (73) to the MRI device (1); and wherein the recommendation signal (73) is configured to adjust a parameter setting of the MRI device (1).

3. The system (100) of claim 1 or claim 2,
wherein the trained MLA (55) is further configured and trained to receive, as a further input, at least one additional piece of information (72), and to generate the recommendation signal (73) based also on said at least one additional piece of information (72).

4. The system (100) of claim 3, wherein the at least one additional piece of information (72) is a piece of information indicating a location of an organ of interest within the received series (71).

5. The system (100) of any of claims 1 to 4,
wherein the trained MLA (55) is a trained artificial neural network.

6. The system (100) of any of claim 5,
wherein the trained MLA (55) is a trained artificial deep neural network.

7. A computer-implemented method for operating a magnetic resonance imaging, MRI, device, comprising:
receiving (S1) a series (71) of MR images obtained at the same slice position with different resonance frequency setting values, RFSV;
providing (S2) a trained machine learning algorithm, MLA, (55) configured and trained to receive, as an input, such a series (71) of MR images, and further configured and trained to generate, as an output, a recommendation signal (73) indicating an optimal RFSV based at least on said input; inputting (S3) the received series (71) of MR images into the provided trained machine learning algorithm, MLA (55); generating (S4), by the provided trained MLA (55), the recommendation signal (73) based on the input series; and outputting (S5) the recommendation signal (S73).

8. The method of claim 7,
wherein the method comprises a step of adjusting (S6) a parameter setting of the MRI device (1) based on the recommendation signal (73).

9. A computer program product (200) comprising executable program code (250) configured to, when executed, perform the method according to any of claim 7 or 8.

10. A non-transitory, computer-readable data storage medium (300) comprising executable program code (350) configured to, when executed, perform the method according to any of claim 7 or 8.

11. A computer-implemented method for training a machine learning algorithm, MLA, (55) comprising:
providing (S10) training samples, wherein each training sample comprises a series (71) of MR images obtained at the same slice position for at least two different resonance frequency setting values and each training sample further comprises a label indicating which image of the series of images best fulfils a given quality criterion;
providing (S20) a machine learning algorithm, MLA, (55) configured to receive said training samples as an input and to output based thereon a recommendation signal (73) indicating an optimal RSFV value, wherein the machine learning algorithm, MLA, (55) comprises trainable parameters;
training (S30) the MLA (55) by updating the trainable parameters based on a loss function penalizing differences of an output of the machine learning algorithm, MLA, (55) for a given training sample and the label of the same training sample.

12. The method of claim 11,
wherein the provided trained MLA (55) is further configured to receive, as a further input, at least one additional piece of information (72), and to generate the output based also on said at least one additional piece of information (72); wherein each training sample further comprises the at least one additional piece of information (72).

13. The method of claim 12, wherein the at least one additional piece of information (72) is a piece of information indicating a location of an organ of interest within the input series.

14. A computer program product (200) comprising executable program code (250) configured to, when executed, perform the method according to any of claims 11 to 13.

15. A non-transitory, computer-readable data storage medium (300) comprising executable program code (350) configured to, when executed, perform the method according to any of claims 11 to 13.
